Europäisches Patentamt

European Patent Office

Office européen des brevets

(19)

(11) Publication number: **0 062 974**
**B1**

(12) **EUROPEAN PATENT SPECIFICATION**

(45) Date of publication of patent specification: **30.07.86**

(51) Int. Cl.⁴: **A 61 M 5/14**

(21) Application number: **82301406.3**

(22) Date of filing: **18.03.82**

(54) **Medication injection device.**

(30) Priority: **10.04.81 US 252779**

(43) Date of publication of application:
**20.10.82 Bulletin 82/42**

(45) Publication of the grant of the patent:
**30.07.86 Bulletin 86/31**

(84) Designated Contracting States:
**DE FR GB IT**

(56) References cited:
**EP-A-0 018 649**
**EP-A-0 019 817**
**WO-A-80/01755**
**WO-A-81/00888**
**US-A-4 142 524**

(73) Proprietor: **PARKER HANNIFIN CORPORATION**
**17325 Euclid Avenue**
**Cleveland Ohio 44112 (US)**

(72) Inventor: **Mayfield, William B.**
**1939 Tustin Avenue**
**Costa Mesa California 92627 (US)**

(74) Representative: **Thomas, Roger Tamlyn et al**
**D. Young & Co. 10 Staple Inn**
**London WC1V 7RD (GB)**

Courier Press, Leamington Spa, England.

**Description**

The invention relates generally to liquid medication injection devices. As hereinafter described, the invention can provide a miniature extracorporeal medication injection device for precisely and conveniently dispensing controlled quantities of a liquid medicine into a human or animal body.

Liquid medications (which, for purposes of this patent application, means any liquid that is to be injected into a human or animal body for therapeutic or diagnostic purposes) have been traditionally administered by a hypodermic syringe. If multiple doses of the liquid medication are required, the multiple doses are typically administered at timed intervals of several days or several hours.

More recently, it has been recognized that the benefits of certain liquid medications may be enhanced by administering the liquid medication in extremely small dosages on a frequent basis. Medication infusion devices which have been designed for this purpose are disclosed in United States Patents Nos. 4,191,181; 4,150,672; 4,215, 689 and 3,858,581. United States Patent No. 4,201,207 discloses a device which administers a constant flow rate of liquid medication.

European specification 0 018 649 discloses a device in which a reservoir for liquid is connected to an outlet by a fluid feed line which includes an elongate tube of elastic material containing unidirectional valve members and defining a pumping chamber and a pulse detector chamber. Clamp elements and pivoted armatures act on the outer walls of the portions of the elongate tube forming the chambers to effect and detect a pumping action. The reservoir and fluid feed line can be replaced when the reservoir has been emptied.

According to the invention there is provided a liquid medication injection device comprising a variable volume collapsible reservoir, an outlet, a pump disposed between the reservoir and the outlet, and including a pump housing defining a variable volume pump chamber and movable means movable relative to the pump housing for altering the volume of the pump chamber, the movable means including a solenoid armature of electromagnetic material, the device having a reusable portion that includes an electromagnetic solenoid core, a solenoid coil and means for energizing the solenoid coil; and a disposable portion comprising the reservoir, the pump chamber, and the electromagnetic solenoid armature, which electromagnetic solenoid armature contacts a first engagement surface when the solenoid coil is energized and is biased against a second engagement surface when the solenoid coil is de-energized, characterised in that a piston is located in and defines at least a portion of the pump chamber and is secured to and movable with the armature, the piston having a first position defining a minimum volume configuration of the pump chamber when the armature is biased against the second engagement surface and a second position defining a maximum volume configuration of the pump chamber when the armature contacts the first engagement surface, and spring means for resiliently biasing the piston such that the piston is urged toward the first position.

Preferably the reusable portion includes a rigid case, a battery power supply, and a programmable electronic controller. The electromagnetic solenoid coil receives an electrical signal from the controller and provides a magnetic actuating force.

The disposable pump of the disposable portion includes inlet and outlet check valves, and the electromagnetic armature varies the volume of the chamber in response to the magnetic actuating force of the coil.

The disposable portion and the reusable portion each preferably include precise alignment surface means disposed in predetermined positions. When the disposable portion is releasably secured in the case of the reusable portion, the alignment surface means of the disposable portion engage the alignment surface means of the reusable portion. In this manner, the armature of the disposable portion is precisely aligned with and spaced a predetermined distance from the core of the reusable portion. When the electrical signal is provided by the controller to the coil, the magnetic force then provides movement of the armature through this predetermined distance. When the electrical signal is terminated, the spring returns the armature to its rest position.

Thus the movement of the armature and corresponding stroke of the pump will always be the same, when the disposable portions are changed on a periodic basis while the device is being used. Additionally, because the pump utilizes the positive action of the spring to return the pump to the rest position at the completion of each pump cycle, the return stroke and output pressure of the pump is also precisely controlled. The injection device can provide the option to the user of either filling the reservoir from a separate vial of liquid medication or purchasing the disposable portion in a prefilled condition for maximum convenience and ease of use.

The invention is diagrammatically illustrated by way of example with reference to the accompanying drawings, in which:—

Figure 1 is a perspective view of a liquid medication injection device according to the invention;

Figure 2 is a perspective view of a disposable portion of the device shown in Figure 1;

Figure 3 is an enlarged cross-sectional view taken on line 3—3 in Figure 2;

Figure 4 is a cross-sectional view taken on line 4—4 in Figure 1;

Figure 5 is a perspective view of a spring that is used to maintain alignment of the disposable portion and the reusable portion of the device shown in Figure 1;

Figure 6 is an enlarged view of an inlet check valve shown in Figure 3;

Figure 7 is a still further enlarged perspective view of the check valve shown in Figure 6;

Figure 8 is an enlarged perspective view of a pump housing of the portion of the device shown in Figures 2 and 3 with the armature shown in phantom;

Figure 9 is a side-elevational view of an armature for the disposable portion shown in Figure 2; and

Figure 10 is an enlarged perspective view of a solenoid core for the reusable portion of the device shown in Figure 4.

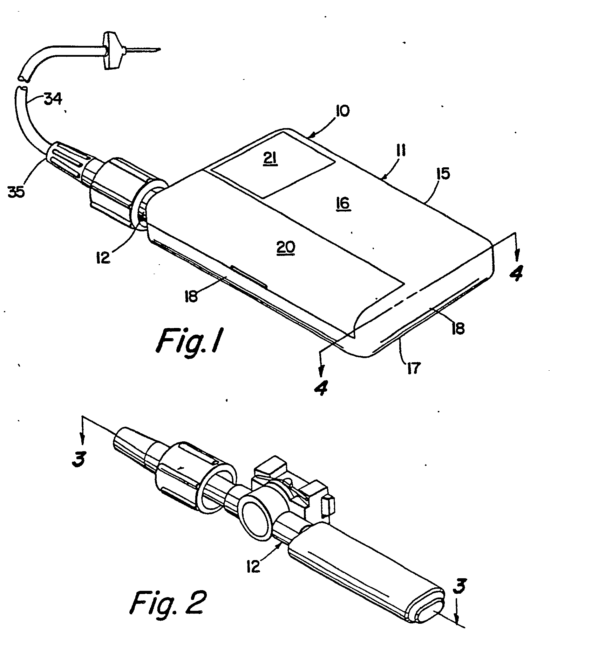

Referring to the drawings, Figure 1 illustrates an extracorporeal liquid medication injection device 10. The medication injection device 10 is of small size (approximately 6.4 cm long, 2.6 cm wide, and 1.3 cm thick) and light weight (approximately 70 gm) and can be carried in the user's pocket or taped or strapped to the user's body. The medication injection device 10, as explained in further detail below, carries a reservoir of liquid medication and provides a means for dispensing the liquid medication into the user's body at a programmed rate.

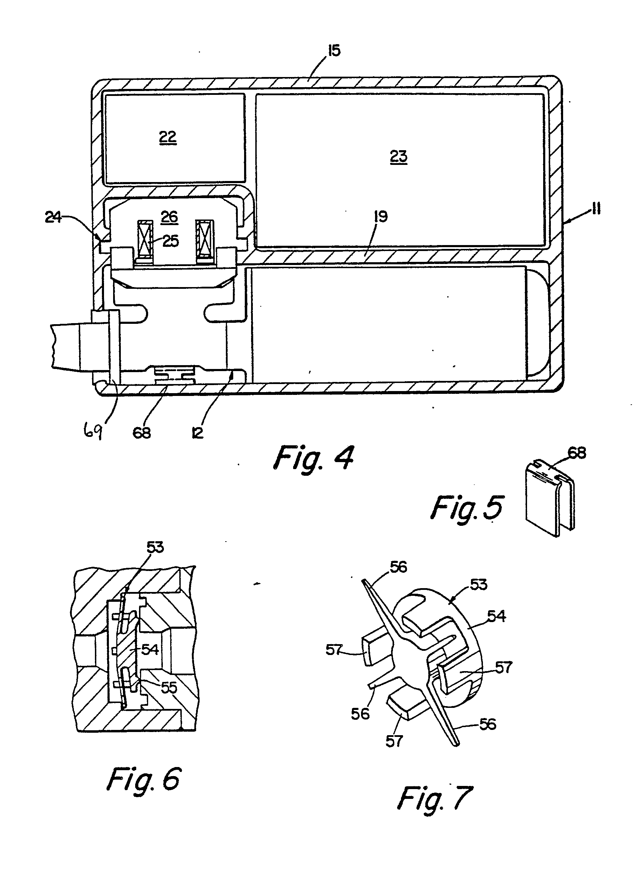

Referring now to Figures 1 and 4 together, the medication injection device 10 includes a reusable portion 11 and a disposable portion 12. The reusable portion 11 can be utilized repeatedly over a period of several years for dispensing liquid medication to the body of the user. The disposable portion 12 can dispense a predetermined quantity of liquid medication which it carries, and is then discarded and replaced with a new disposable portion carrying a new quantity of the liquid medication.

Referring still to Figures 1 and 4, the reusable portion 11 includes a rigid plastics case 15. The case 15 includes a top wall 16, a bottom wall 17, end walls 18, and internal dividing walls 19. The walls 16—19 are all of approximately the same thickness and same material. The top wall 16 includes a removable panel 20 for providing access to the disposable portion 12 and a removable panel 21 for providing access to a battery 22 which provides a source of electrical power for the medication injection device 10. The removable panels 20 and 21 are secured in place by a suitable detent (not shown) in a well known manner.

The reusable portion 11 also includes a programmable electronic controller 23 which receives electrical power from the battery 22 and which provides electrical energy to a solenoid 24.

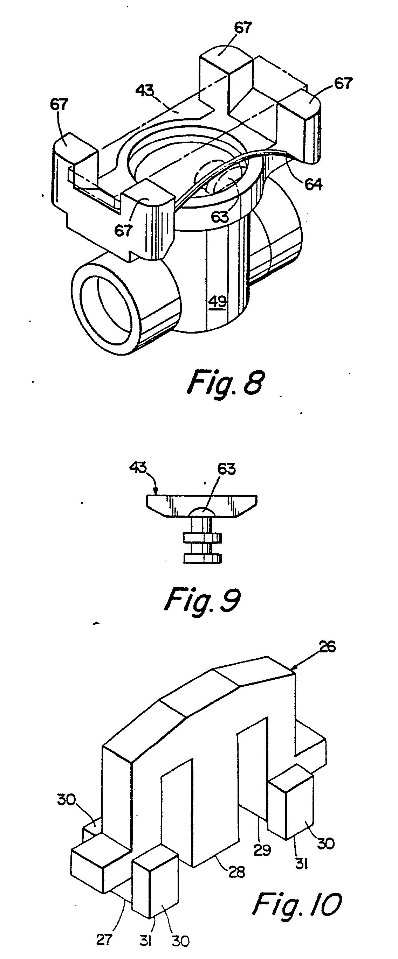

The solenoid 24 includes a coil 25 which is energized by the controller 23 on a programmed basis and an electromagnetic core 26. The core 26, as shown in greater detail in Figure 10, is made of magnetic material (such as a martensitic stainless steel) and is of a generally E-shaped configuration. The core 26 includes end walls 27, 28 and 29 which face toward an armature as explained in greater detail below. The end walls 27 and 29 are co-planar, and the end wall 28 may be recessed slightly from the plane in which the walls 27 and 29 are disposed to facilitate release of the armature after actuation of the coil 25.

Four identical alignment bars 30, only three of which may be seen in Figure 10, are secured at similar locations on opposite sides of the core 26. The alignment bars 30 are each of non-magnetic material (such as an austenitic stainless steel) and are welded to the core 26 in the position shown in Figure 10. The alignment bars 30 each include a precise alignment surface 31, and the alignment surfaces 31 are co-planar. The plane in which the alignment surfaces 31 are disposed is spaced a predetermined distance from the plane in which the core surfaces 27 and 29 are disposed, and the perpendicular distance between these two planes is the stroke of the pump, as explained in further detail below.

Referring back to Figure 1, the medication injection device 10 also includes a replaceable needle and a connecting tube assembly 34. The assembly 34 is connected to the disposable portion 12 by a threaded connection 35. The needle and connecting tube assembly 34 may therefore be disconnected from the disposable portion 12 when the disposable portion 12 is to be replaced.

Referring now to Figures 1, 2 and 3 together, the disposable portion 12 includes a reservoir 41, a pump 42, and an electromagnetic armature 43. The reservoir 41 includes a collapsible plastics bag 44 that is secured within a cylindrical plastics housing 45 and sealingly attached to a plastics end wall 46. The collapsible bag 44 is filled by injecting the liquid medicine that is to be dispensed through a septum 47.

The pump 42 includes a pump housing 49 which defines an inlet passage 50, an outlet passage 51, and a pump chamber 52. The pump 42 also includes check valves 53 placed in the inlet and outlet passages 50 and 51. The check valves 53, as shown in greater detail in Figures 6 and 7, are of one-piece plastics construction and include a base 54. In the preferred embodiment, the check valves are of a thermoplastic carbonate linked polymer such as that sold under the trademark "Lexan" by General Electric Co., or of an acrylic polymer resin such as that sold under trademark "Cryolite G-20" by Cry/Ro Industries. An annular seat 55 projects from one side of the base 54 to provide sealing engagement with an adjacent surface in the passage. A plurality of leaf spring portions 56 project from a central hub of the base 54 and provide a spring force to urge the seat 55 against its adjacent surface. A plurality of axially extending stops 57, which are disposed between adjacent ones of the springs 56, project from the base 54 in a direction opposite to the seat 55 to control the travel of the seat 55 away from its adjacent surface when the check valve 53 is opened.

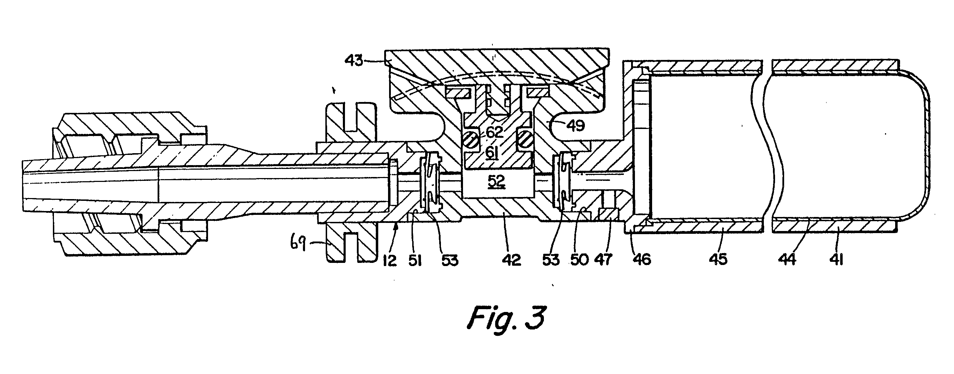

Referring now to Figures 3, 8 and 9 together, the pump 42 further includes a piston 61 which reciprocates in a bore that intersects the pump chamber 52 in a direction perpendicular to the axis of the passages 50 and 51. The piston 61 is of plastics construction and is sealed against the walls of the bore in which it is disposed by an O-ring seal 62. The O-ring seal 62 is dimensioned and arranged so that it is compressed in the groove in the outer peripheral surface of the piston 61 and so that it does not slide relative to either the piston 61 or the bore. Instead, the O-ring 62 is of sufficient elasticity, and the movement of the piston 61 is sufficiently small, that elastic deformation of the O-ring 62 is sufficient to accommodate the full stroke of the piston 61 in the bore.

The electromagnetic armature 43, which is of magnetic material such as martensitic stainless steel, includes a stem portion projecting from its bottom surface which is received with an interference fit in a bore in the piston 61. In this manner, the armature 43 is rigidly connected to the piston 61 so that movement of the armature 43 causes corresponding movement of the piston 61. Two suitable spring rests 63, only one of which is shown in Figures 8 and 9, project laterally in opposite directions from the armature 43. Identical leaf springs 64 are provided on each lateral side of the pump housing 49 and act between the pump housing 49 and the spring rests 63 to urge the piston 61 to its rest position shown in the drawings.

As best shown in Figure 8, the pump housing 49 also includes alignment surfaces 67. The alignment surfaces 67 are co-planar with one another and are co-planar with the top surface of the armature 43. When the pump 42 is manufactured, this co-planar relationship may be accomplished by initially making the alignment surfaces 67 project beyond the top surface of the armature 43, and then, after the armature 43 and springs 64 are installed in the pump housing 49, melting or grinding the surfaces 67 to a co-planar relationship with the top surface of the armature 43.

Referring now to Figures 3 and 4 together, the disposable portion 12 is releasably assembled in the case 15 by opening the removable panel 20. The disposable portion 12 is then inserted into the case 15 in a manner such that the alignment surfaces 67 of the pump housing 49 (Figure 8) contact the alignment surfaces 31 of the core 26 (Figure 10). This contact is established and maintained by a suitable leaf spring 68 (see Figures 4 and 5). An elastomeric grommet 69, which is frictionally received in an open ended slot in the case 15 when the cover 20 is removed, secures the removable portion 12 against axial movement relative to the reusable portion 11.

The materials for reusable portion 11 and disposable portion 12 are selected from commercially available thermoplastic materials to provide adequate strength and rigidity, resistance to chemicals, and ease of manufacturing. In the preferred embodiment, the case 15 is of an acrylonitrile-butadiene-styrene copolymer (ABS), the reservoir 44 is of a suitable flexible thermoplastic urethane resin, and the pump housing 49 and piston 61 are of an acrylic polymer resin such as that sold under the trademark "Cryolite G-20" by Cy/Ro Industries.

When it is desired to pump the liquid medication from the collapsible reservoir 44 to the outlet passage 51 and to the needle and tube assembly 34, the controller 23 provides an electrical current to energize the coil 25. This causes the armature 43 to move upwardly as viewed in the drawings until the top surface of the armature 43 engages the surfaces 27 and 29 of the core 26. This causes the piston 61 to move upwardly to increase the volume of the chamber 52. As the volume of the chamber 52 increases, the check valve 53 in the inlet passage 50 opens to permit liquid medication to flow from the reservoir 44 into the pump chamber 52. When this electrical current is interrupted, the coil 25 is de-energized and the springs 64 push the armature 43 and piston 61 downwardly as viewed in the drawings to decrease the volume of the chamber 52. This causes the check valve 53 in the outlet passage 51 to open to permit flow of the liquid medicine from the pump chamber 52 to the needle and tube assembly 34. When the liquid medication in the reservoir 44 is nearly depleted, the user opens the removable panel 20, removes the existing disposable portion 12, and inserts a new disposable portion 12 with a full reservoir of liquid medication.

**Claims**

1. A liquid medication injection device comprising a variable volume collapsible reservoir (41), an outlet (51), a pump (42) disposed between the reservoir (41) and the outlet (51), and including a pump housing (49) defining a variable volume pump chamber (52) and movable means (26, 43, 61) movable relative to the pump housing (49) for altering the volume of the pump chamber (52), the movable means including a solenoid armature (43) of electromagnetic material, the device having a reusable portion (11) that includes an electromagnetic solenoid core (26), a solenoid coil (25) and means for energizing the solenoid coil (25); and a disposable portion (12) comprising the reservoir (41), the pump chamber (52), and the electromagnetic solenoid armature (43), which electromagnetic solenoid armature (43) contacts a first engagement surface (27, 29) when the solenoid coil (25) is energized and is biased against a second engagement surface when the solenoid coil (25) is de-energized, characterised in that a piston (61) is located in and defines at least a portion of the pump chamber (52) and is secured to and movable with the armature (43), the piston (61) having a first position defining a minimum volume configuration of the pump chamber (52) when the armature (43) is biased against the second engagement surface and a second position defining a maximum volume configuration of the pump chamber (52) when the armature (43) contact the first engagement surface (27, 29), and spring means (64) for resiliently biasing the piston (61) such that the piston (61) is urged toward the first position.

2. A liquid medication injection device according to claim 1, wherein the first engagement surface (27, 29) comprises an end wall of the core (26).

3. A liquid medication injection device according to claim 1 or claim 2, wherein

the reusable portion (11) includes;

a housing (15) with a solenoid compartment and a disposable portion compartment; and

means are provided for releasably securing the disposable portion (12) to the reusable portion (11), the releasable securing means including a spring (68) that co-operates with the disposable portion compartment to bias the disposable portion (12) against the reusable portion (11) to control the stroke length of the armature (43).

4. A liquid medication injection device according to claim 3, wherein the disposable portion (12) and the reusable portion (11) each include alignment surface means (67, 31), and said alignment surface means of said disposable portion engage said alignment surface means of said reusable portion.

5. A liquid medication injection device according to claim 4, wherein the spring (68) of the releasable securing means resiliently biases the alignment surfaces (67, 31) against one another.

6. A liquid medication injection device according to claim 1, wherein the reusable portion includes a first alignment surface means (31) for alignment of the disposable insert (12), and the disposable insert (12) includes a first check valve (53) disposed between the reservoir (41) and the pump chamber (52);

a second check valve (53) disposed between the pump chamber (52) and the outlet (51); and

a second alignment surface means (67) disposed in a predetermined position relative to the second engagement surface and co-operating with the first alignment surface means (31) accurately to position the second engagement surface with respect to the first engagement surface (27, 29).

7. A medication injection device according to claim 6, wherein the second alignment surface means is disposed on the pump housing (49).

**Patentansprüche**

1. Injektionseinrichtung zur Medikation mit einer Flüssigkeit, mit einem volumenveränderbaren, zusammendrückbaren Behälter (41), einem Auslaß (51), einer zwischen dem Behälter (41) und dem Auslaß (51) angeordneten Pumpe (42), die ein Pumpengehäuse (49) besitzt, das eine Förderkammer (52) mit veränderbarem Volumen begrenzt, und relativ zu dem Pumpengehäuse bewegbare Mitteln (26, 43, 61) zum Verändern des Volumens der Förderkammer (52), wobei die bewegbaren Mittel einen aus elektromagnetischem Werkstoff bestehenden Anker (43) eines Elektromagneten umfassen, die Vorrichtung einen wiederholt verwendbaren Teil (11) besitzt, der einen elektromagnetischen Kern (26) eines Elektromagneten umfaßt, ferner eine Magnetspule (25) und eine Einrichtung zum Speisen der Magnetspule (25), und einen nach dem Gebrauch wegzuwerfenden Teil (12), der den Behälter (41), die Förderkammer (52) und den elektromagnetischen Anker (43) umfaßt, der bei stromdurchflossener Magnetspule (25) an einer ersten Anlagefläche (27, 29) angreift und der bei stromloser Magnetspule (25) gegen eine zweite Anlagefläche vorbelastet ist, dadurch gekennzeichnet, daß die Förderkammer (52) mindestens zum Teil von einem darin angeordneten Kolben (61) begrenzt ist, der an dem Anker (43) befestigt und mit ihm bewegbar ist und der bei gegen die zweite Anlagefläche vorbelastetem Anker (43) eine erste Stellung einnimmt, in der die von ihm begranzte Förderkammer (52) ein minimales Volumen besitzt, und der bei an der ersten Anlagefläche (27, 29) angreifendem Anker (43) eine zweite Stellung einnimmt, in der die von ihm begrenzte Förderkammer (52) ein maximales Volumen hat, und daß eine Federeinrichtung (64) zum elastischen Vorbelasten des Kolbens (61) zu seiner ersten Stellung hin vorgesehen ist.

2. Injektionsvorrichtung zur Medikation mit einer Flüssigkeit nach Anspruch 1, dadurch gekennzeichnet, daß die erste Anlagefläche (27, 29) mindestens teilweise von einer Stirnfläche des Kerns (26) gebildet wird.

3. Injektionsvorrichtung zur Medikation mit einer Flüssigkeit nach Anspruch 1 oder 2, dadurch gekennzeichnet, daß der wiederholt verwendbare Teil (11) ein Gehäuse (15) mit einem Abteil für die Magnetspule und einem Abteil für den nach dem Gebrauch wegzuwerfenden Teil aufweist und Mittel zum lösbaren Befestigen des nach dem Gebrauch wegzuwerfenden Teils (12) an dem wiederholt verwendbaren Teil (11) vorgesehen sind und eine Feder (68) umfassen, die mit dem Abteil für den nach dem Gebrauch wegzuwerfenden Teil derart zusammenwirkt, daß sie den nach dem Gebrauch wegzuwerfenden Teil (12) gegen den wiederholt verwendbaren Teil (11) vorbelastet, um die Hublänge des Ankers (43) zu bestimmen.

4. Injektionsvorrichtung zur Medikation mit einer Flüssigkeit nach Anspruch 3, dadurch gekennzeichnet, daß sowohl der nach dem Gebrauch wegzuwerfende Teil (12) als auch der wiederholt verwendbare Teil (11) je eine Paßfläche (67, 31) besitzen und die Paßfläche des nach dem Gebrauch wegzuwerfenden Teils an der Paßfläche des wiederholt verwendbaren Teils angreift.

5. Injektionsvorrichtung zur Medikation mit einer Flüssigkeit nach Anspruch 4, dadurch gekennzeichnet, daß die Feder (68) der Mittel zum lösbaren Befestigen die Paßflächen (67, 31) gegeneinanderdrückt.

6. Injektionsvorrichtung zur Medikation mit einer Flüssigkeit nach Anspruch 1, dadurch gekennzeichnet, daß der wiederholt verwendbare Teil eine erste Paßfläche (31) zum Fluchten des nach dem Gebrauch wegzuwerfenden Einsatzes (12) besitzt und daß der nach dem Gebrauch wegzuwerfende Einsatz (12) ein erstes Rückschlagventil (53) aufweist, das zwischen dem Behälter (41) und der Förderkammer (52) angeordnet ist, ferner ein zweites Rückschlagventil (53), das zwischen dem Förderkammer (52) und dem Auslaß (51) angeordnet ist, und eine zweite Paßfläche (67), die in einer vorherbestimmten Stellung relativ zu der zweiten Anlagefläche angeordnet ist und mit der ersten Paßfläche (31) derart zusammenwirkt, daß die zweite Anlagefläche gegenüber der ersten Anlagefläche (27, 29) genau positioniert ist.

7. Injektionsvorrichtung nach Anspruch 6, dadurch gekennzeichnet, daß die zweite Paßfläche an dem Pumpengehäuse (49) vorgesehen ist.

## Revendications

1. Dispositif pour l'injection de médicaments liquides, comprenant un réservoir déformable à volume variable (41), un orifice de sortie (51), une pompe (42) disposée entre le réservoir (41) et l'orifice de sortie (51), et comprenant un carter de pompe (49) définissant une chambre de pompe à volume variable (52) et des moyens mobiles (26, 43, 61) déplaçables par rapport au carter de pompe (49) pour modifier le volume de la chambre de pompe (52), les moyens mobiles comprenant une armature de solénoïde (43) en matériau électromagnétique, le dispositif comportant une partie réutilisable (11) comprenant un noyau de solénoïde électromagnétique (26), une bobine de solénoïde (25) et des moyens d'excitation de la bobine de solénoïde (25); et une partie jetable (12) comprenant le réservoir (41), la chambre de pompe (52), et l'armature de solénoïde électromagnétique (43, cette armature de solénoïde électromagnétique (43) venant en contact avec une première surface d'engagement (27, 29) lorsque la bobine de solénoïde (25) est excitée, et se trouvant poussée contre une seconde surface d'engagement lorsque la bobine de solénoïde (25) n'est pas excitée, dispositif caractérisé en ce qu'un piston (61) est logé dans la chambre de pompe (52) et définit une partie au moins de celle-ci, et en ce que ce piston (61) est fixé à l'armature (43) pour se déplacer avec celle-ci, le piston (61) comportant une première position définissant une configuration de volume minimum de la chambre de pompe (52) lorsque l'armature (43) est poussée contre la seconde surface d'engagement, et une seconde position définissant une configuration de volume maximum de la chambre de pompe (52) lorsque l'armature (43) vient en contact avec la première surface d'engagement (27, 29), et des moyens de ressort (64) poussant élastiquement le piston (61) de façon que ce piston (61) soit poussé vers le haut en direction de la première position.

2. Dispositif pour l'injection de médicaments liquides selon la revendication 1, caractérisé en ce que la première surface d'engagement (27, 29) est constituée par une paroi d'extrémité du noyau (26).

3. Dispositif pour l'injection de médicaments liquides selon l'une quelconque des revendications 1 et 2, caractérisé en ce que la partie réutilisable (11) comprend:
— carter (15) muni d'un compartiment de solénoïde et d'un compartiment de partie jetable; et
— des moyens permettant de monter, de manière amovible, la partie jetable (12) sur la partie réutilisable (11), ces moyens de montage amovible comprenant un ressort (68) coopérant avec le compartiment de partie jetable pour pousser la partie jetable (12) contre la partie réutilisable (11) pour commander la longueur de course de l'armature (43).

4. Dispositif pour l'injection de médicaments liquides selon la revendication 3, caractérisé en ce que la partie jetable (12) et la partie réutilisable (11) comprennent chacune de moyens de surface d'alignement (67, 31) et en ce que les moyens de surface d'alignement de la partie jetable viennent en contact avec les moyens de surface d'alignement de la partie réutilisable.

5. Dispositif pour l'injection de médicaments liquides selon la revendication 4, caractérisé en ce que le ressort (68) des moyens de montage amovible pousse élastiquement les surfaces d'alignement (67, 31) l'une contre l'autre.

6. Dispositif pour l'injection de médicaments liquides selon la revendication 1, caractérisé en ce que la partie réutilisable comprend des premiers moyens de surface d'alignement (31) destinés à l'alignement de l'élément d'insertion jetable (12), et en ce que l'élément d'insertion jetable (12) comprend un premier clapet de retenue (53) placé entre le réservoir (41) et la chambre de pompe (52); en ce qu'un second clapet de retenue (53) est placé entre la chambre de pompe (52) et la sortie (51); et en ce que des seconds moyens de surface d'alignement (67) sont placés dans une position prédéterminée par rapport à la second surface d'engagement de manière à coopérer avec les premiers moyens de surface d'alignement (31) pour placer avec précision la seconde d'engagement par rapport à la première surface d'engagement (27, 29).

7. Dispositif pour l'injection de médicaments liquides selon la revendication 6, caractérisé en ce que les seconds moyens de surface d'alignement sont placés sur le carter de pompe (49).

*Fig. I*

*Fig. 2*

Fig. 3

**Fig. 4**

**Fig. 5**

**Fig. 6**

**Fig. 7**

Fig. 8

Fig. 9

Fig. 10